(19) Europäisches Patentamt / European Patent Office / Office européen des brevets

(11) **EP 4 517 606 A1**

(12) **EUROPEAN PATENT APPLICATION**
published in accordance with Art. 153(4) EPC

(43) Date of publication:
**05.03.2025 Bulletin 2025/10**

(21) Application number: **22940334.0**

(22) Date of filing: **29.04.2022**

(51) International Patent Classification (IPC):
**G06N 10/60** (2022.01)     **G06N 10/20** (2022.01)
**B82Y 10/00** (2011.01)

(52) Cooperative Patent Classification (CPC):
**G06N 10/00; G06N 10/20; G06N 10/60;
G16C 10/00; G16C 20/20; G16C 20/50;
G16C 60/00;** B82Y 10/00

(86) International application number:
**PCT/KR2022/006137**

(87) International publication number:
**WO 2023/210840 (02.11.2023 Gazette 2023/44)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **28.04.2022 KR 20220052872**

(71) Applicant: **Qunova Computing Inc.
Daejeon 34051 (KR)**

(72) Inventors:
• **KANG, Doo Hyung
Daejeon 35210 (KR)**
• **CHOI, Minjin
Daejeon 34185 (KR)**
• **KIM, Gyeongsil
Pocheon-si Gyeonggi-do 11176 (KR)**
• **LEE, Gwonhak
Suwon-si Gyeonggi-do 16417 (KR)**
• **FUKS, Johanna I.
CABA, 1425 (AR)**

(74) Representative: **Mammel und Maser
Patentanwälte
PartG mbB
Tilsiter Straße 3
71065 Sindelfingen (DE)**

(54) **METHOD FOR CALCULATING ELECTRONIC STRUCTURE OF MATERIAL BY USING QUANTUM COMPUTING**

(57) The present invention relates to a method for calculating an electronic structure of a material by using quantum computing. Particularly, the method of the present invention for calculating an electronic structure of a material performed linked with a quantum computer and a classical computer, may comprise the steps of: degrading a target molecule into fragments of a plurality of monomers having a predetermined positional relationship; performing a first VQE routine for performing a Hamiltonian matrix calculation for the plurality of monomers on the basis of a calculation of electron density for the plurality of monomers, inputting modified electron density, and repeating the first VQE routine performing the Hamiltonian matrix calculation until the modified electron density according to the Hamiltonian matrix by the result of the first VQE routine converges; and performing a second VQE routine for performing a Hamiltonian matrix calculation on one or more dimers consisting of two monomer pairs of the plurality of monomers.

# FIG. 1

DIVIDE TARGET MOLECULE INTO MONOMER FRAGMENTS —S110

CALCULATE ELECTRON DENSITIES FOR MONOMERS —S120

PERFORM HAMILTONIAN MATRIX CALCULATIONS FOR MONOMERS USING VQE —S130

CONVERGENCE OF ELECTRON DENSITIES? S140

NO

YES

PERFORM HAMILTONIAN MATRIX CALCULATIONS FOR DIMERS USING VQE —S150

CALCULATE GROUND-STATE TOTAL ENERGY —S160

## Description

Technical Field

The present invention relates to a method for calculating the electronic structure of a material and, particularly, to a method for calculating the electronic structure of a material by employing the fragment molecular orbital- variational quantum eigensolver (FMO-VQE) method.

Background

In material-related research including physics, biology, and chemistry, much effort has been made to reveal electronic structure and density distributions of materials. The rationale is that the physical properties of materials can be predicted based on their electronic structures. For example, the electronic structures of human proteins can be analyzed for the development of new drugs, the electronic structures of solid materials can be studied for designing new materials, and the electronic structures of catalysts can be used to predict and optimize chemical reactions.

However, considerable resources and time are required by conventional classical computing to calculate the ground-state energy eigenvalues of the Hamiltonian (equation) for elucidating such electronic structures of materials. Quantum computing technology allows for the computation of quantum mechanical energies and electronic structures of materials through exponentially fewer computational resources compared with classical computing.

The Fragment Molecular Orbital (FMO) method elucidates the electronic structure and electron density distribution of a material by dividing the system into fragments, enabling the calculation of much larger systems. FMO was first presented in the work by Kitaura et al., "Fragment molecular orbital method: an approximate computational method for large molecules," Chemical Physics Letters, 1999, 313, 701, which is incorporated in the references.

The Variational Quantum Eigensolver (VQE) method on the other hand, was designed for calculating the ground-state energy eigenvalues of electronic Hamiltonians by combining classical and quantum computing. The hybrid quantum-classical VQE method is described in McClean et al., "The theory of variational hybrid quantum-classical algorithms," New Journal of Physics, 2016, 18, 023023, which is incorporated in the references.

In the present invention, a method is proposed for rapidly and accurately calculating the ground-state energy of an electronic Hamiltonian. The approach aims to elucidate the electronic structure of a material by integrating the Fragment Molecular Orbital (FMO) method with the Variational Quantum Eigensolver (VQE) technique.

Disclosure of Invention

Technical Problem

The goal of the present invention is to provide a method for calculating the electronic structure of a material by employing fragment molecular orbital-variational quantum eigensolver (FMO-VQE) method, which combines the FMO method with the VQE method.

Solution to Problem

The presented method for calculating the electronic structure of a material is implemented by a classical computer (CC) interworking with a quantum computer (QC). The method includes: dividing a target molecule into multiple monomer fragments; executing a first VQE routine on the Hamiltonian matrix calculations on the multiple monomers on the basis of the calculation of electron densities for the multiple monomers, iterating until the convergence of electron densities corrected according to Hamiltonian matrixes resulting from the first VQE routine is achieved; and executing a second VQE routine of performing Hamiltonian matrix calculations on one or more dimers each consisting of a pair of two monomers among the multiple monomers.

The method includes calculating the ground-state total energy of the target molecule according to Hamiltonian matrices resulting from the second VQE routine.

In the first VQE routine, the Hamiltonian matrices are obtained by iterating the run of quantum circuits using the quantum state on the quantum computer and the parameter updating according to the results of the run on the classical computer.

In the first VQE routine, Hamiltonian matrix $H_I$ for the I-th monomer among the multiple N monomers is obtained by solving the equation,

$$H_I = \sum_i^{n_I} \left\{ -\frac{1}{2} \nabla_i^2 - \sum_s^{\text{all atoms}} \frac{Z_s}{|r_i - r_s|} + \sum_{J \neq I}^{N} \int dr' \frac{\rho_J(r')}{|r_i - r'|} \right\} + \sum_{i>j}^{n_I} \frac{1}{|r_i - r_j|}$$

where: $n_I$ is the number of all electrons in the I-th monomer; $-\frac{1}{2}\nabla_i^2$ is the kinetic energy of the i-th electron; $r_i$ and $r_j$ are the position vectors of electrons; $r_s$ is the position of nucleus s among all atoms in the monomer; $Z_s$ is the nuclear charge of the corresponding nucleus s; J is the index of a monomer other than monomer I, and $\rho_J(r')$ is the electron density at position r' in monomer J.

In the first VQE routine, the electron density $\rho_J(r')$ at position r' in the monomer J is approximated to the average electron density.

In the second VQE routine, the Hamiltonian matrices are obtained by iterating the run of quantum circuits using the quantum state on the quantum computer and the parameter updating according to the results of the run on the classical computer.

In the second VQE routine, Hamiltonian matrix $H_{IJ}$ for the corresponding dimer corresponding to a pair of the I-th and J-th monomers among the multiple N monomers is obtained based on the calculation of equation,

$$H_{IJ} = \sum_i^{n_I + n_J} \left\{ -\frac{1}{2} \nabla_i^2 - \sum_s^{\text{all atoms}} \frac{Z_s}{|r_i - r_s|} + \sum_{K \neq I,J}^{N} \int dr' \frac{\rho_K(r')}{|r_i - r'|} \right\} + \sum_{i>j}^{n_I + n_J} \frac{1}{|r_i - r_j|}$$

where: $n_I$ and $n_J$ each are the number of all electrons in each of the corresponding monomers constituting the dimer; $-\frac{1}{2}\nabla_i^2$ is the kinetic energy of the i-th electron; $r_i$ and $r_j$ are the position vectors of electrons; $r_s$ is the position of nucleus s among all atoms in the dimer; $Z_s$ is the nuclear charge of the corresponding nucleus s; K is the index of a dimer other than the monomer pair (I, J); and $\rho_K(r')$ is the electron density at position r' in dimer K.

In the second VQE routine, the electron density $\rho_K(r')$ at position r' in the dimer K is approximated to the average electron density.

The ground-state total energy E of the target molecule is calculated based on the Schrödinger equation,

$$H_I \Psi_I = E_I' \Psi_I$$

$$H_{IJ} \Psi_{IJ} = E_{IJ}' \Psi_{IJ}$$

($H_I$ and $H_{IJ}$ are Hamiltonians for the monomer and the dimer, $\Psi_I$ and $\Psi_{IJ}$ are wave functions of the monomer and the dimer, and $E_I'$ and $E_{IJ}'$ are energies of the monomer and the dimer), and is obtained by solving the equation,

$$E = \sum_{I>J}^{N} E_{IJ}' - (N-2) \sum_I^N E_I' + \sum_{s>t}^{\text{all atoms}} \frac{Z_s Z_t}{|r_s - r_t|}$$

where: N is the number of monomers, I and J are the monomer indexes; $r_s$ and $r_t$ are the positions of nucleuses s and t among all atoms of the target molecule; and $Z_s$ and $Z_t$ are the nuclear charges of the corresponding nucleuses s and t.

Advantageous Effects of Invention

The present invention describes a method to calculate the electronic structure of a material, through a combination of the FMO method, which can simulate large systems through fragmentation, and the VQE method, which calculates the ground-state energy eigenvalues of the Hamiltonian using a quantum computer. The Hamiltonian matrix calculations for monomers are iterated until convergence using the VQE method, followed by Hamiltonian matrix calculations for dimers, leading to a useful method for analyzing electronic structures and density distributions of materials.

Brief Description of Drawings

The accompanying drawings, which are included as a part of the description to help the understanding of the invention, together with the description, explain the technical aspects of the invention.

FIG. 1 is a flowchart for illustrating the method of calculating the electronic structure of a material using the FMO-VQE procedure presented in this invention.
FIG. 2 illustrates the division of a system into monomers and dimers according to the FMO method.
FIG. 3 illustrates the VQE method for calculating the ground-state energy eigenvalue of an electronic Hamiltonian combining classical and quantum computing.
FIG. 4 illustrates the iteration procedure of the Hamiltonian matrix calculations of monomers by a combination of the restricted Hartree-Fock FMO (FMO-RHF) method on a classical computer (CC) and the VQE method combining classical computing (CC) and quantum computing (QC), according to the present invention.
FIG. 5 illustrates an example of the performance of the FMO-VQE method for an H2 polymer molecule, compared to the traditional RHF and FMO-RHF methods. The y-axis shows the error w.r.t. to the exact Full Configuration Interaction (FCI) energy (in Hartrees).
FIG. 6 illustrates the H2 polymer molecule analyzed in FIG. 5, where d1 is the interatomic distance between the H atoms in the H2 molecule and d2 is the inter-fragment distance.
FIG. 7 is an analysis of the H2 hexamer molecule energy as a function of the distance d2, comparing the performance of the FMO-VQE method presented in this invention against RHF and FMO-RHF.
FIG. 8 illustrates the six monomers constituting the H2 hexamer molecule analyzed in FIG. 7.

Best way to carry out the Invention

The present invention will now be described in detail with reference to the accompanying drawings. Throughout the respective drawings, the same reference labels are used for the same elements if possible. The detailed descriptions of already known functions and/or configurations are omitted. The following description emphasizes the essential components needed to understand the operation of the method, while omitting details of elements that may obscure the main subject matter. In addition, some elements of the drawings may be exaggerated, omitted, or schematically illustrated. The size of each element does not entirely reflect the actual size, and thus the contents described herein are not limited by the relative size or spacing of the elements drawn in the respective drawings.

Firstly, we describe the classical computer (CC) and the quantum computer (QC) used in the present invention.

The classical computer (cc) used in the present invention may be composed of hardware, software, or a combination thereof, and for example, the classical computer may be implemented as a server or computing system having at least one processor for performing the above functions. Such a computing system may include at least one processor, memory, user interface input device, user interface output device, storage, and network interface, which are connected via a bus. The processor may be a central processing unit (CPU) or a semiconductor device that processes instructions stored in the memory and/or storage. The memory and storage may include various types of volatile or nonvolatile storage media. For example, the memory may include a read only memory (ROM) and a random access memory (RAM). The network interface may include a communication module such as a modem that supports wired Internet communication, wireless Internet communication, such as WiFi, WiBro, and the like, mobile communication such as WCDMA, LTE, and the like in a user equipment, such as a smartphone, a laptop PC, a desktop PC, and the like, or may include a communication module such as a modem that supports communication based on a short-range wireless communication scheme (e.g., Bluetooth, Zigbee, WiFi, and the like). Therefore, the steps of a method or algorithm described in connection with the method disclosed herein may be materialized directly in hardware, in a software module executed by a processor, or in a combination of the two. A software module may reside in a storage medium (that is, a memory and/or a storage), such as RAM memory, flash memory, ROM memory, EPROM memory, EEPROM memory, registers, a hard disk, a removable disk, or a CD-ROM. An external storage medium may be coupled to the processor, such that the processor can read information from, and write information to, the storage medium. Alternatively, the storage medium may be integral to the processor. The processor and the storage medium may reside in an application-specific integrated circuit (ASIC). The ASIC may reside in a user terminal. Alternatively, the processor and the storage medium may reside as discrete components in a user terminal.

The quantum computer (QC) is a quantum computing device that performs calculations by using quantum mechanical phenomena, such as exponential information representation through quantum superposition and parallel computation using quantum entanglement. The classical computer (CC) is a binary digital electronic computer based on transistors and capacitors, where data is represented and computed as bits, but the quantum computer (QC) represents and computes data as qubits, qutrits, or qudits, which may be in a quantum state of superposition (e.g., the polarization direction of photons). The quantum computer (QC) utilizing such quantum-state information processing technology can perform multiple calculations simultaneously in a superposed manner within a single processing unit, thereby improving the information processing capacity and speed by over 1000 times compared with the classical computer (CC). A description of the method presented in this invention follows, wich aims to calculate the electronic structure of a material using the fragment molecular orbital-variational quantum eigensolver (FMO-VQE) procedure, which combines the FMO method processed on a classical computer (CC) and the VQE method using the interworking of a classical computer (CC) and a quantum computer (QC). The FMO method is used for analyzing a material by using fragments composed of monomers and dimers, and the VQE method is used for calculating the ground-state energy eigenvalue of the Hamiltonian through quantum computing on a quantum computer (QC).

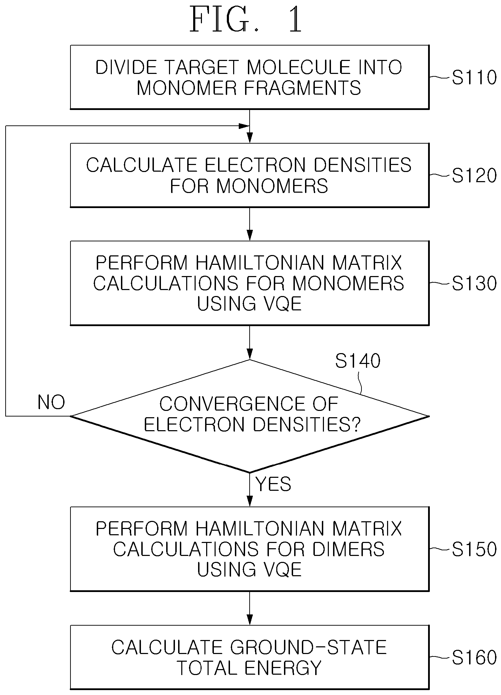

FIG. 1 is a flowchart illustrating the present invention aimed at calculating the electronic structure of a material using the FMO-VQE method.

Referring to FIG. 1, in the method for calculating the electronic structure of a material according to the present invention, which is implemented by a classical computer (CC) interworking with a quantum computer (QC), a target molecule of the corresponding material is first divided into multiple monomer fragments (M1, M2, ...) (S110). The target molecule may be constituted by various chemical materials, such as proteins, new materials, and catalysts, and may be a set of atoms, ions, or molecules.

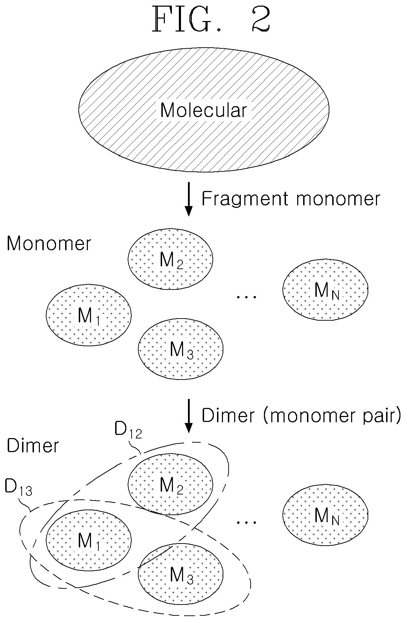

FIG. 2 illustrates the material fragments subject to electronic structure calculation according to the present invention.

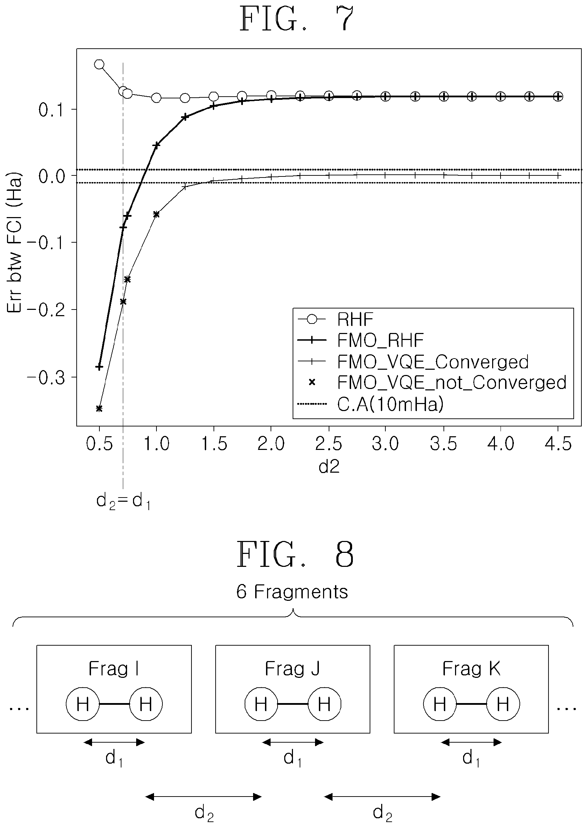

As shown in FIG. 2, the target molecule of the material may be divided into multiple monomer fragments (M1, M2, ...) with a predetermined positional relationship, and may include one or more dimers (D12, D13, ...) each consisting of a pair of two monomers among the multiple monomers (M1, M2, ...). The monomer fragments (M1, M2, ...) may be atoms, ions, molecules, or conjugates thereof. FIG. 8 exemplifies a target molecule consisting of six H2 monomer fragments (Frag I, J, K, ...), showing relative positional relationships, such as d1, the distance between atoms H-H constituting each monomer, and d2, the distance between dimers each consisting of an H2 pair.

Then, the electron densities for the multiple monomers (M1, M2, ...) having a predetermined positional relationship in the target molecule are calculated (e.g., by the RHF method) (S120), and a first VQE routine of performing Hamiltonian matrix calculations on the multiple monomers according to the input of the calculated electron densities is executed (S130). Steps S120 to S140 illustrated in Fig 1 are repeated until the convergence of the electron densities corrected based on the Hamiltonian matrices from the first VQE routine (S140) is achieved. This involves re-inputting the corrected electron densities (S120) and executing the first VQE routine to perform the Hamiltonian matrix calculations (S130).

The electron density distributions of the monomers are calculated on the classical computer (CC). The equations for this calculation are well-known in physics or chemistry through the Hartree-Fock method or the like, and thus detailed descriptions will be omitted herein.

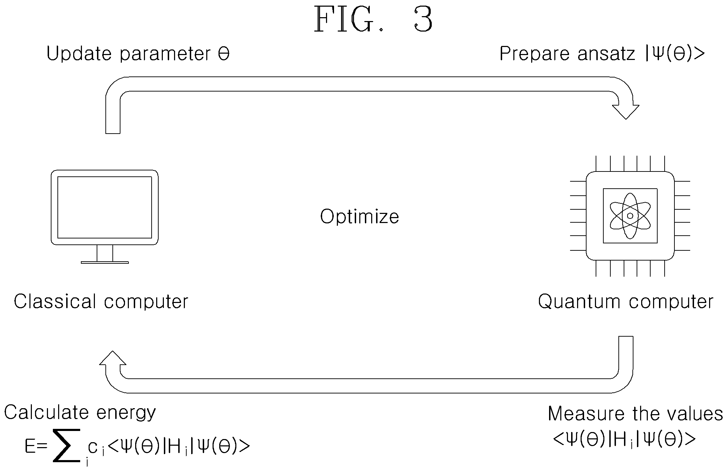

FIG. 3 illustrates the VQE method for calculating the ground-state energy eigenvalue of the Hamiltonian by quantum computing according to the present invention.

In FIG. 3, the first VQE routine (S130) which performs the Hamiltonian matrix calculation is illustrated. The quantum state $\langle\Psi(\Theta)|H_i|\Psi(\Theta)\rangle$ is calculated based on the Ansatz, an initial hypothesis used to solve the problem by establishing the solution's form. The quantum state is prepared on the quantum computer (QC), and a quantum circuit is run (e.g., the calculation of the quantum state $\langle\Psi(\Theta)|H_i|\Psi(\Theta)\rangle$ associated with the Hamiltonian matrix). The energy $E = \Sigma_i c_i \langle\Psi(\Theta)|H_i|\Psi(\Theta)\rangle$ is measured on the quantum computer (QC), and the Ansatz parameters ($\theta$) are updated accordingly. A new energy estimate is computed based on the corrected parameters ($\theta$) on the classical computer and this procedure is iterated until convergence is reached. I $\rho_J(r')$ at position r' in the monomer J is approximated to the average electron density.

In other words, in the first VQE routine (S130), through the interworking of the classical computer (CC) and the quantum computer (QC), the Hamiltonian matrix $H_I$ for the I-th monomer among the multiple N monomers (M1, M2, ...) is obtained based on the calculation of [Equation 1].

[Equation 1]

$$H_I = \sum_i^{n_I} \left\{ -\frac{1}{2}\boldsymbol{\nabla}_i^2 - \sum_s^{\text{all atoms}} \frac{Z_s}{|r_i - r_s|} + \sum_{J \neq I}^{N} \int dr' \frac{\rho_J(r')}{|r_i - r'|} \right\} + \sum_{i>j}^{n_I} \frac{1}{|r_i - r_j|}$$

where: $n_I$ is the number of all electrons in the I-th monomer; $-\frac{1}{2}\boldsymbol{\nabla}_i^2$ is the kinetic energy of the i-th electron of monomer I; $r_i$ and $r_j$ are the position vectors of electrons included in the I-th monomer; $r_s$ is the position of nucleus s among all atoms in the monomer; $Z_s$ is the nuclear charge of the corresponding nucleus s; J is the index of a monomer other than monomer I; and $\rho_J(r')$ is the electron density at position r' in monomer J.

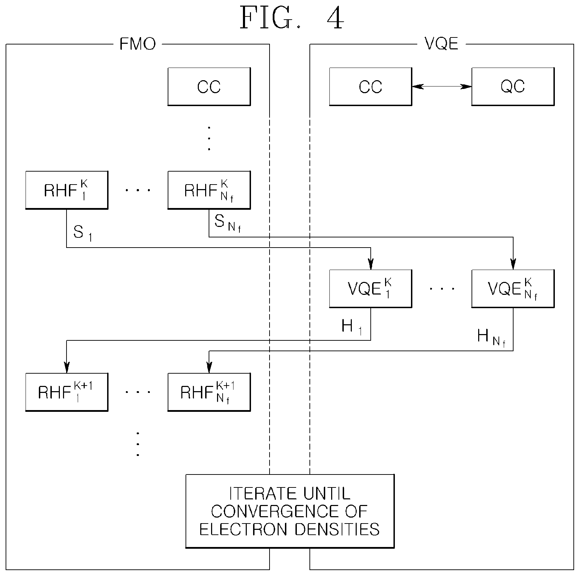

FIG. 4 illustrates the iteration procedure of Hamiltonian matrix calculations for monomers by a combination of the FMO method and the VQE method according to the present invention.

Referring to FIG. 4, in the procedure of iterating Hamiltonian matrix calculations until the convergence of electron densities in steps S120 to S140, the present invention is implemented by combining the FMO method and the VQE method yielding the FMO-VQE method. In the FMO method, on the classical computer (CC), comparatively simple calculations RHF1K to RHFNFK, such as calculations of electron densities for Nf monomers based on the Hartree-Fock calculation or the like, are performed. Then the information S1 to SNf about the results is delivered; and in the VQE method, on the quantum computer (QC), interworking with the classical computer (CC), the ansatz for the Hamiltonian matrix calculation using the quantum state from the delivered information is established. The Hamiltonian matrix calculations VQE1K to VQENFK through corresponding quantum circuits are performed using the same, and iterative updating is performed according to the resultant H1 to HNf until the convergence of the electron densities, thereby generating a resultant Hamiltonian matrix that serves as the basis for the ground-state energy eigenvalue estimate. In the present invention, the application of the fragment molecular orbital-variational quantum eigensolver (FMO-VQE) to the calculation of the electronic structure of a material enables the quantum computer (QC), interworking with the classical computer (CC), to quickly perform the Hamiltonian matrix calculations for monomers until the convergence of electron densities via quantum computing, even though the number of iterations increases. Therefore, the method of the present invention can promptly perform Hamiltonian matrix calculations for dimers and can provide an advantageous manner to analyze the electronic structure and density distribution of a material.

Next, a second VQE routine is performed, on the classical computer (CC), Hamiltonian matrix calculations for one or more dimers each consisting of a pair of two monomers among the multiple monomers (M1, M2, ...) according to the convergence result of Hamiltonian matrix calculations for all the monomers in steps S120 to S140 is executed (S150).

In the second VQE routine (S150) of performing Hamiltonian matrix calculations, the VQE method as shown in FIG. 3 is performed, i.e. an ansatz is chosen for Hamiltonian matrix calculation using the quantum state on the quantum computer (QC) (e.g., the calculation of the quantum state $\langle \Psi(\Theta)|H_i|\Psi(\Theta)\rangle$ associated with the Hamiltonian matrix based on the ansatz, etc.), followed by the measurement of the energy $E = \Sigma_i c_i \langle \Psi(\Theta)|H_i|\Psi(\Theta)\rangle$ and the updating of parameter ($\theta$) for correction of the ansatz on the basis of the calculation are iterated one or more times to obtain Hamiltonian matrices. In the second VQE routine (S150), the electron density $\rho_K(r')$ at position r' in the dimer K is approximated to the average electron density.

In other words, in the second VQE routine (S150), through the interworking of the classical computer (CC) and the quantum computer (QC), the Hamiltonian matrix $H_{IJ}$ for the corresponding dimer consisting of a pair of the I-th and J-th monomers among the multiple N monomers may be obtained on the basis of the calculation of [Equation 2].

[Equation 2]

$$H_{IJ} = \sum_{i}^{n_I + n_J} \left\{ -\frac{1}{2} \nabla_i^2 - \sum_{s}^{all\ atoms} \frac{Z_s}{|r_i - r_s|} + \sum_{K \neq I,J}^{N} \int dr' \frac{\rho_K(r')}{|r_i - r'|} \right\} + \sum_{i>j}^{n_I + n_J} \frac{1}{|r_i - r_j|}$$

where: $n_I$ and $n_J$ each are the number of all electrons in each of the corresponding monomers constituting the dimer;

$$-\frac{1}{2} \nabla_i^2$$

is the kinetic energy of the i-th electron; $r_i$ and $r_j$ are the position vectors of electrons; $r_s$ is the position of nucleus s among all atoms in the dimer; $Z_s$ is the nuclear charge of the corresponding nucleus s; K is the index of a dimer other than the monomer pair (I, J); and $\rho_K(r')$ is the electron density at position r' in dimer K.

Next, upon the completion of the Hamiltonian matrix calculations for dimers in the second VQE routine (S150), the ground-state total energy of the target molecule is computed according to the Hamiltonian matrices by the results of the second VQE routine in the classical computer (CC) (S160).

That is, the ground-state total energy E of the target molecule is calculated on the basis of the Schrödinger equation as shown in Equation 3.

[Equation 3]

$$H_I \Psi_I = E_I' \Psi_I$$

$$H_{IJ} \Psi_{IJ} = E_{IJ}' \Psi_{IJ}$$

In the equation, $H_I$ and $H_{IJ}$ are Hamiltonians for the monomer and the dimer; $\Psi_I$ and $\Psi_{IJ}$ are wave functions of the monomer and the dimer; and $E_I'$ and $E_{IJ}'$ are energies of the monomer and the dimers.

Therefore, the ground-state total energy E of the target molecule is obtained from Equation 4.

[Equation 4]

$$E = \sum_{I>J}^{N} E_{IJ}' - (N-2) \sum_{I}^{N} E_I' + \sum_{s>t}^{all\ atoms} \frac{Z_s Z_t}{|r_s - r_t|}$$

In the equation, N is the number of monomers; I and J are the monomer indexes; $r_s$ and $r_t$ are the positions of nucleuses s and t among all atoms in the target molecule; and $Z_s$ and $Z_t$ are the nuclear charges of nucleuses s and t.

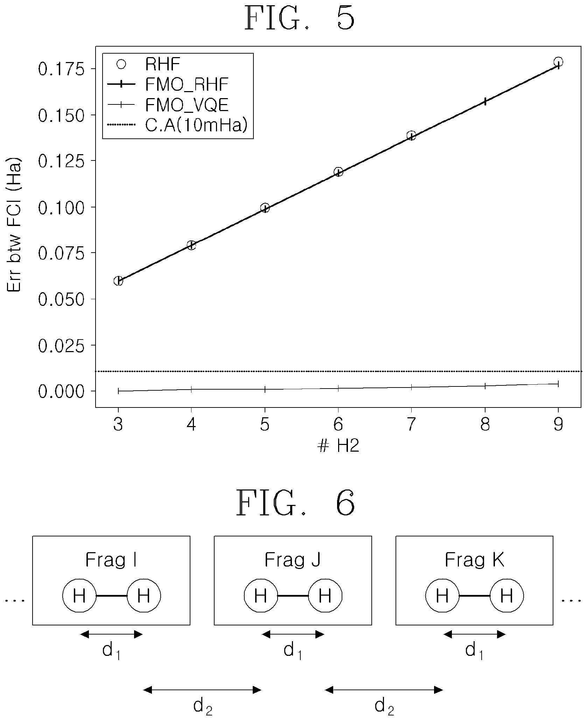

FIG. 5 illustrates an example of the performance of the FMO-VQE method for an H2 polymer molecule. The y-axis shows the error w.r.t. to the exact Full Configuration Interaction (FCI) energy (in Hartrees). FMO-VQE achieves chemical accuracy (C.A.) for all polymer lengths, while the error for FMO-RHF increases for longer polymers.

FIG. 6 illustrates the H2 polymer molecule analyzed in FIG. 5, where d1 is the interatomic distance between the H atoms in the H2 molecule and d2 is the inter-fragment distance.

In FIG. 5, the results for the traditional Hartree-Fock method (RHF), the general combined FMO method and Hartree-Fock method (FMO_RHF), and the FMO-VQE combined method of the present invention (FMO VQE) as function of the number of H2 molecules in the H2 polymer are presented and the error is computed w.r.t. the energy of the full configuration interaction (FCI) method (CA 10 mHA), which serves as a reference for computational chemistry.

In FIGS. 5 and 6, the quantum computer calculation as performed using the full orbital space in the STO-3G basis, using 4 qubits per monomers, and 8 qubits per dimers for ditances d1=0.7237 Å and d2=2.9506 Å.

As shown in FIG. 5, the traditional Hartree-Fock method (RHF) and the combined general FMO method and Hartree-Fock method (FMO_RHF) show errors significantly larger than CA (10 mHa), and they increase with increasing number (#H2) of H2 monomer fragments (Frag I, J, K, ...), but the combined FMO-VQE method (FMO_VQE) of the present invention shows smaller errors than CA 10 mHa for all #H2, indicating significant improvement w.r.t to RHF and FMO-RHF.

FIG. 7 is an analysis of the H2 hexamer molecule energy as a function of the distance d2 between the H2 fragments, comparing the performance of the FMO-VQE method presented in this invention against RHF and FMO-RHF. FIG. 8 illustrates the six monomers constituting the H2 hexamer molecule analyzed in FIG. 7.

In FIG. 7 the errors of the traditional Hartree-Fock method (RHF), the general combined FMO method and Hartree-Fock method (FMO_RHF), and the FMO-VQE combined method of the present invention (FMO_VQE) were compared with the results of the full configuration interaction (FCI) method (CA 10 mHA), which serves as a reference for computational chemistry, as a function of the distance (d2) in the H2 hexamer.

Referring to FIGS. 7 and 8, the calculation on the quantum computer was made using the full orbital space in the STO-3G basis set, using 4 qubits for monomers, and 8 qubits for dimers for d1=0.7237 Å.

As shown in FIG. 7, the traditional Hartree-Fock method (RHF) and the combined general FMO method and Hartree-Fock method (FMO_RHF) show significantly larger errors than CA 10 mHa even though the distance (d2) in the target molecule consisting of six H2 monomer fragments (Frag I, J, K, ...) was d2 = 1.5 d1 or more. The combined FMO-VQE method (FMO_VQE) of the present invention shows significantly improved results, smaller errors than CA 10 mHa at d2 = 1.5 d1, with some non-converged results within d2 = 1.0 d1.

According to the method of the present invention for calculating the electronic structure of a material (FMO-VQE), through a combination of the FMO method, which analyzes a material fragmented into monomers and dimers, and the VQE method, which calculates the ground-state energy eigenvalue of the Hamiltonian by quantum computing. In this approach, Hamiltonian matrix calculations for monomers are iteratively performed using the VQE method until electron density convergence is achieved. Subsequently, Hamiltonian matrix calculations for dimers are conducted based on the converged Hamiltonian matrices. This process enables rapid Hamiltonian matrix calculations for monomers to achieve electron density convergence via quantum computing, followed by swift Hamiltonian matrix calculations for dimers. The method is valuable for analyzing the electronic structures and density distributions of materials.

## Claims

1. A method for calculating the electronic structure of a material, which is implemented by a classical computer (CC) interworking with a quantum computer (QC), the method comprising:

   dividing a target molecule into multiple monomer fragments having a predetermined positional relationship;
   executing a first VQE routine of performing Hamiltonian matrix calculations on the multiple monomers, this routine is iterated until the convergence of electron densities corrected according to the Hamiltonian matrixes resulting from the first VQE routine is reached; and
   executing a second VQE routine of performing Hamiltonian matrix calculations on one or more dimers each consisting of a pair of two monomers among the multiple monomers.

2. The method of claim 1, further comprising calculating the ground-state total energy of the target molecule according to Hamiltonian matrices resulting from the second VQE routine.

3. The method of claim 1, wherein in the first VQE routine, the Hamiltonian matrices are obtained by iterating the run of quantum circuits using the quantum state on the quantum computer and the parameter updating according to the results of the run on the classical computer.

4. The method of claim 1, wherein in the first VQE routine, Hamiltonian matrix $H_I$ for the I-th monomer among the multiple N monomers is obtained on the basis of the calculation of equation,

$$H_I = \sum_i^{n_I} \left\{ -\frac{1}{2} \nabla_i^2 - \sum_s^{\text{all atoms}} \frac{Z_s}{|r_i - r_s|} + \sum_{J \neq I}^{N} \int dr' \frac{\rho_J(r')}{|r_i - r'|} \right\} + \sum_{i>j}^{n_I} \frac{1}{|r_i - r_j|}$$

where: $n_I$ is the number of all electrons in the I-th monomer; $-\dfrac{1}{2}\boldsymbol{\nabla}_i^2$ is the kinetic energy of the i-th electron; $r_i$ and $r_j$ are the position vectors of electrons; $r_s$ is the position of nucleus s among all atoms in the monomer; $Z_s$ is the nuclear charge of the corresponding nucleus s; J is the index of a monomer other than monomer I, and $\rho_J(r')$ is the electron density at position r' in monomer J.

5. The method of claim 4, wherein in the first VQE routine, the electron density $\rho_J(r')$ at position r' in the monomer J is approximated to the average electron density.

6. The method of claim 1, wherein in the second VQE routine, the Hamiltonian matrices are obtained by iterating the run of quantum circuits using the quantum state on the quantum computer and the parameter updating according to the results of the run on the classical computer.

7. The method of claim 1, wherein in the second VQE routine, Hamiltonian matrix $H_{IJ}$ for the corresponding dimer corresponding to a pair of the I-th and J-th monomers among the multiple N monomers is obtained on the basis of the calculation of equation,

$$H_{IJ} = \sum_i^{n_I + n_J}\left\{ -\frac{1}{2}\boldsymbol{\nabla}_i^2 - \sum_s^{\text{all atoms}} \frac{Z_s}{|r_i - r_s|} + \sum_{K \neq I,J}^{N} \int dr' \frac{\rho_K(r')}{|r_i - r'|}\right\} + \sum_{i>j}^{n_I + n_J} \frac{1}{|r_i - r_j|}$$

where: $n_I$ and $n_J$ each are the number of all electrons in each of the corresponding monomers constituting the dimer;

$$-\frac{1}{2}\boldsymbol{\nabla}_i^2$$

is the kinetic energy of the i-th electron; $r_i$ and $r_j$ are the position vectors of electrons; $r_s$ is the position of nucleus s among all atoms in the dimer; $Z_s$ is the nuclear charge of the corresponding nucleus s; K is the index of a dimer other than the monomer pair (I, J); and $\rho_K(r')$ is the electron density at position r' in dimer K.

8. The method of claim 7, wherein in the second VQE routine, the electron density $\rho_K(r')$ at position r' in the dimer K is approximated to the average electron density.

9. The method of claim 2, wherein the ground-state total energy E of the target molecule is calculated on the basis of the Schrödinger equation,

$$H_I \Psi_I = E_I' \Psi_I$$

$$H_{IJ} \Psi_{IJ} = E_{IJ}' \Psi_{IJ}$$

($H_I$ and $H_{IJ}$ are Hamiltonians for the monomer and the dimer, $\Psi_I$ and $\Psi_{IJ}$ are wave functions of the monomer and the dimer, and $E_I'$ and $E_{IJ}'$ are energies of the monomer and the dimer), and is obtained by the calculation of equation,

$$E = \sum_{I>J}^{N} E_{IJ}' - (N-2)\sum_{I}^{N} E_I' + \sum_{s>t}^{\text{all atoms}} \frac{Z_s Z_t}{|r_s - r_t|}$$

where: N is the number of monomers, I and J are the monomer indexes; $r_s$ and $r_t$ are the positions of nucleuses s and t among all atoms of the target molecule; and $Z_s$ and $Z_t$ are the nuclear charges of the corresponding nucleuses s and t.

**10.** A classical computer interoperating with the quantum computer to implement the method for calculating the electron structure of a material of claim 1.

**11.** A quantum computer interoperating with the classical computer to implement the method for calculating the electron structure of a material of claim 1.

__y__

EP 4 517 606 A1

# FIG. 1

DIVIDE TARGET MOLECULE INTO MONOMER FRAGMENTS —S110

CALCULATE ELECTRON DENSITIES FOR MONOMERS —S120

PERFORM HAMILTONIAN MATRIX CALCULATIONS FOR MONOMERS USING VQE —S130

S140 — CONVERGENCE OF ELECTRON DENSITIES?

NO

YES

PERFORM HAMILTONIAN MATRIX CALCULATIONS FOR DIMERS USING VQE —S150

CALCULATE GROUND-STATE TOTAL ENERGY —S160

12

# FIG. 2

# FIG. 3

Update parameter θ       Prepare ansatz $|\Psi(\theta)\rangle$

Optimize

Classical computer       Quantum computer

Calculate energy

$$E=\sum_i c_i \langle\Psi(\theta)|H_i|\Psi(\theta)\rangle$$

Measure the values

$$\langle\Psi(\theta)|H_i|\Psi(\theta)\rangle$$

EP 4 517 606 A1

FIG. 4

## FIG. 5

## FIG. 6

# FIG. 7

# FIG. 8

6 Fragments

| INTERNATIONAL SEARCH REPORT | | International application No.<br><br>**PCT/KR2022/006137** |
|---|---|---|

**A.    CLASSIFICATION OF SUBJECT MATTER**

**G06N 10/60**(2022.01)i; **G06N 10/20**(2022.01)i; B82Y 10/00(2011.01)n

According to International Patent Classification (IPC) or to both national classification and IPC

**B.    FIELDS SEARCHED**

Minimum documentation searched (classification system followed by classification symbols)

G06N 10/60(2022.01); G06J 1/00(2006.01); G16C 10/00(2019.01); G16C 60/00(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 모노머(monomer), 다이머(dimer), 해밀토니안(hamiltonian), 변형 양자 고유 해석기(variational quantum eigensolver, VQE)

**C.    DOCUMENTS CONSIDERED TO BE RELEVANT**

| Category* | Citation of document, with indication, where appropriate, of the relevant passages | Relevant to claim No. |
|---|---|---|
| X<br>A | YAMAZAKI, Takeshi et al. Towards the Practical Application of Near-Term Quantum Computers in Quantum Chemistry Simulations: A Problem Decomposition Approach. arXiv:1806.01305v1. June 2018. [Retrieved on 16 January 2023]. Retrieved from <https://arxiv.org/abs/1806.01305v1>.<br>    See pages 1, 4-7 and 12. | 1-3,6,10-11<br>4-5,7-9 |
| A | PARRISH, Robert M. et al. Quantum Computation of Electronic Transitions using a Variational Quantum Eigensolver. arXiv:1901.01234v2. April 2019. [Retrieved on 16 January 2023]. Retrieved from <https://arxiv.org/abs/1901.01234v2>.<br>    See pages 1-11. | 1-11 |
| A | MALONE, Fionn D. et al. Towards the simulation of large scale protein–ligand interactions on NISQ-era quantum computers. Chemical Science. Vol. 13, No. 11, 21 March 2022.<br>    See pages 3094-3107. | 1-11 |
| A | CN 109859807 A (FUDAN UNIVERSITY) 07 June 2019 (2019-06-07)<br>    See claims 1-7. | 1-11 |

| ✓ Further documents are listed in the continuation of Box C. | ✓ See patent family annex. |
|---|---|

<table>
<tr><td colspan="2">*    Special categories of cited documents:</td><td>"T"</td><td>later document published after the international filing date or priority date and not in conflict with the application but cited to understand the principle or theory underlying the invention</td></tr>
<tr><td>"A"</td><td>document defining the general state of the art which is not considered to be of particular relevance</td><td rowspan="2">"X"</td><td rowspan="2">document of particular relevance; the claimed invention cannot be considered novel or cannot be considered to involve an inventive step when the document is taken alone</td></tr>
<tr><td>"D"</td><td>document cited by the applicant in the international application</td></tr>
<tr><td>"E"</td><td>earlier application or patent but published on or after the international filing date</td><td rowspan="2">"Y"</td><td rowspan="2">document of particular relevance; the claimed invention cannot be considered to involve an inventive step when the document is combined with one or more other such documents, such combination being obvious to a person skilled in the art</td></tr>
<tr><td>"L"</td><td>document which may throw doubts on priority claim(s) or which is cited to establish the publication date of another citation or other special reason (as specified)</td></tr>
<tr><td>"O"</td><td>document referring to an oral disclosure, use, exhibition or other means</td><td>"&"</td><td>document member of the same patent family</td></tr>
<tr><td>"P"</td><td>document published prior to the international filing date but later than the priority date claimed</td><td></td><td></td></tr>
</table>

| Date of the actual completion of the international search | Date of mailing of the international search report |
|---|---|
| **25 January 2023** | **25 January 2023** |

| Name and mailing address of the ISA/KR<br><br>**Korean Intellectual Property Office**<br>**Government Complex-Daejeon Building 4, 189 Cheongsa-ro, Seo-gu, Daejeon 35208** | Authorized officer |
|---|---|
| Facsimile No. **+82-42-481-8578** | Telephone No. |

Form PCT/ISA/210 (second sheet) (July 2019)

**INTERNATIONAL SEARCH REPORT**

| International application No. |
| --- |
| **PCT/KR2022/006137** |

**C.    DOCUMENTS CONSIDERED TO BE RELEVANT**

| Category* | Citation of document, with indication, where appropriate, of the relevant passages | Relevant to claim No. |
| --- | --- | --- |
| A | WO 2021-081641 A1 (THE GOVERNING COUNCIL OF THE UNIVERSITY OF TORONTO et al.) 06 May 2021 (2021-05-06)<br>    See claims 1-34. | 1-11 |

Form PCT/ISA/210 (second sheet) (July 2019)

**INTERNATIONAL SEARCH REPORT**
Information on patent family members

| | International application No. |
|---|---|
| | **PCT/KR2022/006137** |

| Patent document cited in search report | | | Publication date (day/month/year) | Patent family member(s) | | | Publication date (day/month/year) |
|---|---|---|---|---|---|---|---|
| CN | 109859807 | A | 07 June 2019 | CN | 109859807 | B | 30 July 2021 |
| WO | 2021-081641 | A1 | 06 May 2021 | US | 2022-0389414 | A1 | 08 December 2022 |

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KITAURA et al.** Fragment molecular orbital method: an approximate computational method for large molecules. *Chemical Physics Letters*, 1999, vol. 313, 701

- **MCCLEAN et al.** The theory of variational hybrid quantum-classical algorithms. *New Journal of Physics*, 2016, vol. 18, 023023